# EUROPÄISCHE PATENTSCHRIFT

(11) **EP 2 204 199 B1**
(45) Veröffentlichungstag und Bekanntmachung des Hinweises auf die Patenterteilung: **26.10.2011**
(21) Anmeldenummer: 09100006.7
(22) Anmeldetag: 05.01.2009
(51) Int. Cl.: A61L 27/06, A61L 27/30, A61L 27/54, A61L 27/56, A61L 31/02, A61L 31/08, A61L 31/14, A61L 31/16

(54) **Verfahren zur Herstellung einer antiinfektiösen Beschichtung auf Implantaten**
Method for manufacturing an anti-infection coating on implants
Procédé de fabrication d'une couche anti-infectieuse sur des implants

(43) Veröffentlichungstag der Anmeldung: 07.07.2010
(73) Patentinhaber: DOT GmbH, D-18059 Rostock (DE)
(72) Erfinder: Neumann, Hans-Georg, Prof. Dr., 18146 Rostock (DE); Prinz, Cornelia, 18337 Marlow (DE)
(74) Vertreter: Garrels, Sabine

(56) Entgegenhaltungen:
- DE-A1- 10 241 137
- DE-U1- 20 020 649
- US-A- 5 833 463
- US-A1- 2005 060 021

## Beschreibung

Die Erfindung betrifft ein Verfahren zur Herstellung einer antiinfektiösen Beschichtung auf Implantaten, die Titan enthalten oder aus Titan bestehen.

### Stand der Technik

Aus der DE 10243132 B4 ist es bekannt, Implantate aus Titan oder Titanlegierungen unter Verwendung eines Sol-Gel-Verfahrens mit einer Titanoxid-Beschichtung zu versehen, in die homogen Metallionen wie Silber oder Kupfer verteilt sind. Zur mechanischen Stabilisierung und Verdichtung der Beschichtung erfolgt nach dem Trocknen eine Wärmebehandlung. Durch Erhitzen auf etwa 500°C erfolgt eine Keramisierung der Beschichtung. Die Wärmebehandlung hat für die Implantate den Nachteil, dass sie zum Verlust der Festigkeit führt. Sie ist deshalb nicht geeignet für Implantate, die eine hohe Dauerfestigkeit aufweisen müssen.

Es ist bekannt, dass eine anodische Oxidation Typ II bei Titan zu einer größeren Härte und höheren Dauerfestigkeit führt. In der DE 20020649 U1 wird neben Silber und Kupfer diese Beschichtung aufgrund ihrer glatten Oberfläche auch als antiinfektiöse Beschichtung beschrieben. Auf einer anodischen Oxidation Typ II-Beschichtung ist es jedoch nicht möglich, eine ausreichend haftfeste Beschichtung von Silber oder Kupfer herzustellen.

### Darstellung der Erfindung

Der Erfindung liegt die Aufgabe zugrunde, ein Beschichtungsverfahren aufzuzeigen, mit dem es möglich ist, für Implantate aus Titan oder die Titan enthalten die mit der anodischen Oxidation Typ II erreichte Optimierung der mechanischen Eigenschaften mit der der antiinfektiösen Eigenschaften zu vereinen.

Diese Aufgabe wird dadurch gelöst, dass die Implantate in einer alkalischen Lösung anodisch oxidiert werden, anschließend auf dieser Oberfläche Metall, das antiinfektiöse Eigenschaften aufweist, galvanisch abgeschieden und danach die metallhaltige Oxidschicht verfestigt wird.

Bei der anodischen Oxidation kann eine Konversionsschicht, die Sauerstoff und ggf. andere Atome enthält, und eine poröse Titanoxidschicht mit ausreichender Leitfähigkeit gebildet werden, die in den Poren elektrisch leitend ist, so dass in ihr Metall galvanisch abgeschieden werden kann. Durch das Strahlen z.B. mit Glaskugeln wird die metallhaltige Oxidschicht verfestigt und schwächer gebundene Oxidund Metallpartikel entfernt oder intensiver miteinander und mit der Implantatoberfläche verbunden.

Es wird ein Verfahren zur kombinierten Modifizierung von Implantaten, die Titan enthalten oder aus Titan bestehen, bereitgestellt, bei dem im ersten Schritt durch anodische Funkenentladung in stark basischen Elektrolyten eine poröse Oxidschicht gebildet wird, im zweiten Schritt der galvanische Eintrag des Metalls in die poröse Schicht erfolgt und in einem 3. Schritt die metallangereicherte Oxidschicht durch Strahlen verfestigt und mehr oder weniger entfernt werden kann, indem schwächer gebundene Oxid- oder Metallpartikel entfernt oder intensiver miteinander und dem Implantat verbunden werden.

Die unter physiologischen Bedingungen einsetzende Elution des Metalls kann durch die Variation der mittleren Metallbelegung der Implantatoberfläche in Ihrer Konzentration so eingestellt werden, dass eine antimikrobielle bzw., antibakterielle Wirkung ohne wesentliche Schädigung der Zellen des umgebenden Körpergewebe erzielt wird. Als Metalle eignen sich insbesondere Kupfer, Silber und Zink. Im Fall des Metalls Kupfer ist durch die katalytische Wirkung auf die Angiogenese dazu eine bessere Durchblutung des neugebildeten Körpergewebes zu erwarten. Die Dicke der metallhaltigen Schicht beträgt vorteilhaft 8-15 µm, bevorzugt 10 µm.

Das Verfestigen der metallhaltigen Oxidschicht erfolgt vorteilhaft durch Strahlen mit Glaskugeln.

### Kurze Beschreibung der Abbildungen

Fig. 1 Partiell mit Kupfer beschichtete TiAIV-Oberfläche

Fig. 2 Zellzahlentwicklung von Staphylococcus aureus ATCC 25923 nach Kultur auf partiell mit Kupfer beschichteter TiAIV-Oberfläche

### Ausführung der Erfindung

Im Folgenden wird die Erfindung an Hand eines Beispieles näher erläutert.

**Beispiel 1: Beschichtung**

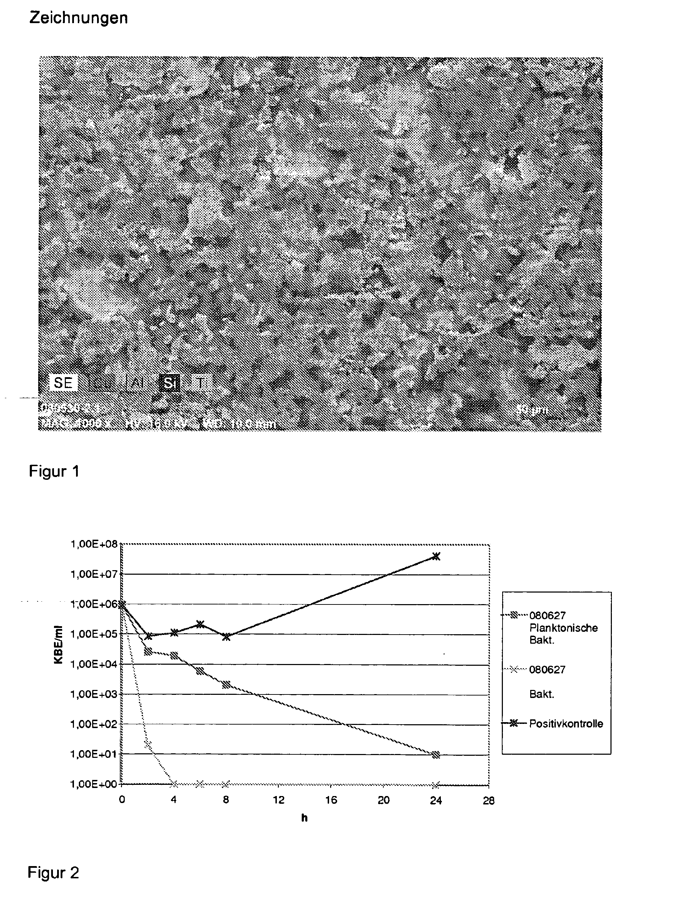

TiAl6V4-Probekörper werden nach dem Prinzip der anodischen Funkenentladung in einem stark basischen Elektrolyten mit einer porösen Oxidschicht überzogen. Dafür werden in 500 ml destilliertes Wasser 50 g NaOH gelöst. In der auf 40°C erwärmten Lösung wird die anodische Funkenoxidation der Titanprobe durch langsames Erhöhen der Spannung auf 40 Volt vorgenommen. Anschließend wird durch kathodische Abscheidung aus einer gesättigten Kupferacetat-Lösung in die poröse Oxidschicht Kupfer eingetragen. Die so mit Kupfer angereicherte Oxidschicht wird durch Glaskugelstrahlen teilweise abgetragen und das sie enthaltene Kupfer in die Probenoberfläche inselartig nahezu völlig eingearbeitet (Fig. 1).

**Beispiel 2: Darstellung der antibakteriellen Wirkungsweise**

Zur Testung der antibakteriellen Wirkung wurden Untersuchungen am klinisch relevanten Bakterienstamm Staphylococcus aureus ATCC25923 durchgeführt. Dafür wurden TiAIV-Zylinder mit einem Durchmesser von 8 mm und einer Länge von 20 mm Länge gemäß Beispiel 1 mit Kupfer beschichtet und in 6ml einer mit 150 µl Bakteriensuspension geimpften PBS-Pufferlösung bei 37 °C unterschiedlich lange ausgelagert. Danach wurde die Konzentration der auf den Zylindern und in der Lösung sich befindenden lebenden Bakterien durch Ausplattieren auf Agar-Nährböden und Auslagern (48 h) bei 30 °C ermittelt. Auf den Zylindern wurden schon nach 4 Stunden keine, in der Puffer-Lösung nach 24 Sunden nur noch sehr wenige lebende Bakterien gefunden (Fig. 2).

## Patentansprüche

1. Verfahren zur Herstellung einer antiinfektiösen Beschichtung auf Implantaten, die Titan enthalten oder aus Titan bestehen, **gekennzeichnet durch** folgende Schritte
- Bildung einer porösen Oxidschicht **durch** anodische Oxidation in einer alkalischen Lösung derart, dass die Leitfähigkeit in den Poren eine galvanische Abscheidung ermöglicht,
- galvanische Abscheidung eines Metalls mit antiinfektiösen Eigenschaften,
- Verfestigung der metallhaltigen Oxidschicht **durch** Strahlen.

2. Verfahren nach Anspruch 1, **dadurch gekennzeichnet, dass** der Elektrolyt für die anodische Oxidation Natronlauge enthält.

3. Verfahren nach Anspruch 1 oder 2, **dadurch gekennzeichnet, dass** der Elektrolyt Natronwasserglas enthält.

4. Verfahren nach Anspruch 1, **dadurch gekennzeichnet, dass** in die poröse Oxidschicht Kupfer, Silber und/oder Zink eingetragen werden.

5. Verfahren nach Anspruch 1, **dadurch gekennzeichnet, dass** die metallhaltige Oxidschicht mit Glaskugeln gestrahlt wird.

6. Verfahren nach Anspruch 1, **dadurch gekennzeichnet, dass** die Oxidschichtdicke 8 - 15 µm beträgt.

## Claims

1. A method for producing an anti-infective coating on implants which contain titanium or are composed of titanium, **characterized by** the following steps:
- formation of a porous oxide layer by anodic oxidation in an alkaline solution such that the conductivity enables an electrodeposition in the pores,
- electrodeposition of a metal with anti-infective properties,
- solidification of said metal-containing oxide layer by blasting.

2. The method according to claim 1, **characterized in that**
the electrolyte for said anodic oxidation contains sodium hydroxide solution.

3. Method according to claim 1 or 2, **characterized in that**
said electrolyte contains soda water glass.

4. The method according to claim 1, **characterized in that**
copper, silver and/or zinc are added into said porous oxide layer.

5. The method according to claim 1, **characterized in that**
said metal-containing oxide layer is blasted with glass beads.

6. The method according to claim 1, **characterized in that**
the thickness of said oxide layer is 8 to 15 µm.

## Revendications

1. Procédé de fabrication d'une couche anti-infectieuse sur des implants qui contiennent du titane ou sont en titane, **caractérisé par** les étapes suivantes:
- formation d'une couche d'oxyde poreuse par oxydation anodique dans une solution alcaline de telle sorte que la conductibilité permet dans les pores une précipitation galvanique,
- précipitation galvanique d'un métal avec propriétés anti-infectieuses,
- durcissement de la couche d'oxyde métallifère par radiation.

2. Procédé selon la revendication 1, **caractérisé en ce que** l'électrolyte pour l'oxydation anodique contient de la soude caustique.

3. Procédé selon la revendication 1 ou 2, **caractérisé en ce que** l'électrolyte contient du silicate de soude.

4. Procédé selon la revendication 1, **caractérisé en ce que** du cuivre, de l'argent et/ou du zinc sont chargés dans la couche d'oxyde poreuse.

5. Procédé selon la revendication 1, **caractérisé en ce que** la couche d'oxyde métallifère est irradiée avec des billes de verre.

6. Procédé selon la revendication 1, **caractérisé en ce que** la couche d'oxyde a une épaisseur de 8 à 15 µm.
